(19) Europäisches Patentamt
European Patent Office
Office européen des brevets

(11) Veröffentlichungsnummer: **0 186 816**
**B1**

(12) # EUROPÄISCHE PATENTSCHRIFT

(45) Veröffentlichungstag der Patentschrift: **13.07.88**

(51) Int. Cl.⁴: **C 07 C 69/708**, C 07 C 67/313, C 07 C 67/31, C 07 C 69/716

(21) Anmeldenummer: **85115645.5**

(22) Anmeldetag: **09.12.85**

(54) **Verfahren zur Herstellung von Glyoxylsäurehemiacetalestern.**

(30) Priorität: **20.12.84 DE 3446528**

(43) Veröffentlichungstag der Anmeldung: **09.07.86 Patentblatt 86/28**

(45) Bekanntmachung des Hinweises auf die Patenterteilung: **13.07.88 Patentblatt 88/28**

(84) Benannte Vertragsstaaten: **AT BE CH DE FR GB IT LI NL**

(56) Entgegenhaltungen: **Keine Entgegenhaltungen**

(73) Patentinhaber: **HOECHST AKTIENGESELLSCHAFT, Postfach 80 03 20, D-6230 Frankfurt am Main 80 (DE)**

(72) Erfinder: **Driscoll, Robert Kenneth, Dr., Heilmann-Strasse 43, D-6000 Frankfurt am Main 50 (DE)**
Erfinder: **Leupold, Ernst Ingo, Dr., Am Zäunefeld 15, D-6392 Neu-Anspach (DE)**
Erfinder: **Ebertz, Wolfgang, Dr., Bismarckstrasse 25, D-5300 Bonn 1 (DE)**

EP 0 186 816 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Glyoxylsäurehemiacetalestern aus Glykolsäureestern.

Aus der US-PS 4 340 748 ist bekannt, dass sich Glykolsäureester an heterogenen Katalysatoren in der Gasphase zu Glyoxylsäureestern oxydehydrieren lassen. Der Reaktoraustrag enthält im wesentlichen Glyoxylsäureester, Wasser, nicht umgesetzten Glykolsäureester, sowie kleine Mengen des von der Estergruppe stammenden Alkohols. Bei der Kondensation solcher Gasgemische entstehen schwer auftrennbare Mischungen aus Hydraten, Hemiacetalen und Acetalen des Glyoxylsäureesters und der Glyoxylsäure.

In der deutschen Patentanmeldung P 33 23 372.1 wird eine Methode zur Herstellung eines Glyoxylsäureesters vorgeschlagen, wobei die unerwünschte Bildung der Folgeprodukte Glyoxylsäureesterhydrate, Glyoxylsäurehemiacetalester, Glyoxylsäureacetalester und Glyoxylsäure verhindert wird, wenn man aus dem gasförmigen Produktgemisch mit einem Schleppmittel das Wasser und den als Nebenprodukt entstandenen Alkohol vor Eintritt der Kondensation abtrennt. Auf diese Weise wird die Bildung eines Hemiacetalesters weitgehend unterdrückt.

Ziel der vorliegenden Erfindung ist dagegen nicht, die Bildung des Hemiacetalesters zu verhindern, sondern ihn herzustellen.

Gegenstand der vorliegenden Erfindung ist daher ein Verfahren zur Herstellung von Glyoxylsäurehemiacetalestern, dadurch gekennzeichnet, dass man Glykolsäureester in der Gasphase katalytisch zu Glyoxysäureestern oxydehydriert und diese dann mit einem Alkohol umsetzt, wobei man das bei der Oxydehydrierung gebildete gasförmige Reaktionsgemisch zusammen mit einem Überschuss eines als Schleppmittel geeigneten Alkohols in eine Destillationskolonne einleitet und dann das Reaktionswasser zusammen mit anderen Leichtsiedern und einem Teil des überschüssigen Alkohols über Kopf destilliert, wobei der Glyoxylsäurehemiacetalester und der Rest des überschüssigen Alkohols als Sumpfprodukt anfallen. Es werden also zunächst die Glykolsäureester katalytisch in der Gasphase zu Glyoxylsäureestern oxydehydriert und diese anschliessend mit Alkoholen zu den entsprechenden Hemiacetalestern umgesetzt:

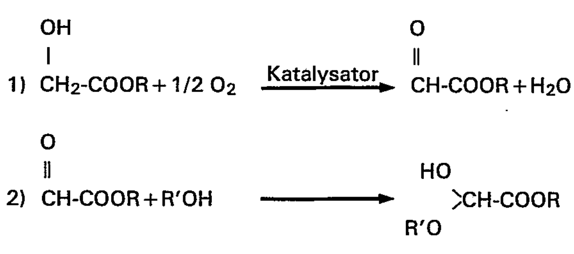


Bei dem erfindungsgemässen Verfahren werden in der ersten Stufe (Oxydehydrierung) Glykolsäureester der allgemeinen Formel $HO\text{-}CH_2\text{-}COOR$ in Dampfform eingesetzt.

Dabei ist R ein Kohlenwasserstoffrest, vorzugsweise ein aliphatischer,geradkettiger oder verzweigter Alkylrest mit 1 bis 8 C-Atomen, insbesondere mit 1 bis 5 C-Atomen.

Die gasförmigen Glykolsäureester werden zusammen mit Sauerstoff oder einem sauerstoffhaltigen Gas, wie Luft, über den Katalysator geleitet. Vorzugsweise verdünnt man mit einem Trägergas, wie Stickstoff oder Egelgas.

Bei dem erfindungsgemässen Verfahren setzt man pro Mol Glykolsäureester im allgemeinen folgende Mengen an Zusatzstoffen ein:

Sauerstoff: 0,1 bis 5 Mol, vorzugsweise 0,5 bis 3 Mol
Trägergas: 0 bis 200 Mol, vorzugsweise 30 bis 100 Mol.

Auch ausserhalb dieser Grenzen werden noch zufriedenstellende Ergebnisse erzielt.

Als Katalysator für das erfindungsgemässe Verfahren kommt im Prinzip jeder Oxydehydrierungskatalysator in Frage. Der Katalysator enthält jedoch vorzugsweise mindestens eines der Elemente V, Au, Mo, Ag, Cu, Sn, Sb, Bi, P. Jedoch zeigen auch andere Elemente der 3. bis 5. Hauptgruppe eine katalytische Wirkung.

Die genannten Elemente werden entweder in metallischer Form oder in Form ihrer Verbindungen, z.B. als Oxide, Nitrate, Acetate, Acetylacetonate, Oxalate, Citrate oder Halogenide in die Reaktionszone eingebracht.

Es hat sich als vorteilhaft erwiesen, vor dem Einleiten des Glykolsäureesters in die Reaktionszone ein oxydierendes Gas, insbesondere Sauerstoff oder Luft, oder ein reduzierendes Gas, insbesondere Wasserstoff oder mit Inertgas verdünnten Wasserstoff, bei Temperaturen von 100 bis 800°C, insbesondere 300 bis 600°C, über den Katalysator zu leiten.

Die katalytisch aktiven Elemente werden vorzugsweise auf Trägermaterialien aufgebracht. Als Träger eignen sich vor allem Silicate, Aluminiumoxide, Aluminiumsilicate, Bimsstein, Siliciumcarbid oder Kohlen. Vorzugsweise verwendet man Silicate, Aluminiumoxide oder Aluminiumsilicate. Besonders vorteilhaft sind Aluminiumsilicate mit einer BET-Oberfläche von weniger als 50 $m^2/g$.

Die Gesamtmenge an Elementen auf dem Träger kann in weiten Grenzen schwanken. Im allgemeinen beträgt sie 0,01 bis 50 Gew.-%, vorzugsweise 0,1 bis 20 Gew.-%, bezogen auf die Gesamtmasse des Trägerkatalysators. Die katalytisch aktiven Komponenten werden zweckmässig in Form einer Lösung auf den Träger gebracht, dann wird das Lösungsmittel abgedampft und der Katalysator getrocknet. Als Lösungsmittel verwendet man im allgemeinen Wasser, Salzsäure, Salpetersäure, Alkalilaugen oder wässrige Ammoniaklösung, vorzugsweise Wasser oder Salzsäure.

Die aktiven Komponenten können jedoch auch ohne Träger eingesetzt werden.

Die Oxydehydrierung wird im allgemeinen bei Temperaturen zwischen 100 und 600°C, vorzugsweise zwischen 200 und 400°C durchgeführt. Die Verweilzeit liegt vorzugsweise zwischen 0,05 und 10 Sekunden, insbesondere jedoch zwischen 0,05 und 1 Sekunde. Auch ausserhalb dieser Grenzen erhält man noch befriedigende Ergebnisse.

Die Oxydehydrierung wird bevorzugt bei Normaldruck durchgeführt, jedoch können auch verminderte oder höhere Drucke angewendet werden, d.h. 0,01 bis 100 bar.

Im einzelnen geht man bei der Oxydehydrierung so vor, dass der Glykolsäureester und Sauerstoff oder sauerstoffhaltiges Gas, sowie ggf. Trägergas, aus Dosierungsvorrichtungen in eine Verdampfungszone und die entstandene Gasmischung dann durch ein von aussen beheiztes und mit dem Katalysator gefülltes Reaktionsrohr geleitet wird. Es hat sich dabei als vorteilhaft erwiesen, den Sauerstoff oder das sauerstoffhaltige Gas sowie das Trägergas vor der Einleitung in die Verdampfungszone auf die Reaktionstemperatur aufzuheizen.

In der zweiten Stufe des erfindungsgemässen Verfahrens wird das gasförmige Reaktionsgemisch aus der Oxydehydrierung nach Verlassen des Reaktionsrohres zusammen mit einem Überschuss eines als Wasser-Schleppmittel geeigneten Alkohols in eine Destillationskolonne eingeleitet. Wasser wird mit einem Teil des überschüssigen Alkohols azeotrop über Kopf entfernt, zusammen mit anderen Leichtsiedern, z.B. Formaldehyd und Methylformiat. Der Sumpf besteht im wesentlichen aus dem gewünschten Glyoxylsäurehemiacetalester, dem Rest des überschüssigen Alkohols, kleinen Mengen des von der Estergruppe stammenden Alkohols sowie unumgeseztem Glykolsäureester. Der gewünschte Glyoxylsäurehemiacetalester kann z.B. durch eine übliche Destillation isoliert werden.

Vorzugsweise wird das Wasser vollständig über Kopf entfernt, um unerwünschte Verseifungsreaktionen im Sumpf zu vermeiden.

Aus dem Kopfprodukt kann der mit dem Wasser überdestillierte Alkohol nach bekannten Methoden, z.B. Destillation oder Phasentrennung gewonnen und zurückgeführt werden.

Es ist bekannt, dass in saurem Medium die Hemiacetale mit Alkoholen weiter zu Vollacetalen reagieren können. Es war ausserdem zu erwarten, dass die Einleitung eines Glyoxylsäureesters in einen Alkohol-Überschuss unter sauren Bedingungen zu Umesterungsreaktionen führen würde. Obwohl kleine Mengen Ameisensäure als Nebenprodukt im Reaktionsgemisch enthalten sind, also saure Bedingungen herrschen, entstehen überraschenderweise beim erfindungsgemässen Verfahren nur geringe Mengen dieser unerwünschten Nebenprodukte.

Nicht alle aliphatischen Alkohole bilden jedoch Azeotrope mit Wasser, eine Ausnahme ist z.B. Methanol. In diesen Fällen arbeitet man mit einem zusätzlichen inerten Schleppmittel.

Ausserdem bilden die Mehrzahl der Alkohole ein Azeotrop mit einem relativ hohen Siedepunkt, nämlich 90 bis 100°C. Da nun viele Glyoxylsäurehemiacetalester sich oberhalb von 90°C zu zersetzen beginnen, ist es bei Einsatz derartiger Alkohole zur Acetalisierung empfehlenswert, als Wasserschleppmittel einen zusätzlichen Stoff einzusetzen, dessen Azeotrop unterhalb von 90°C siedet. Dieses zusätzliche Schleppmittel sollte mit dem Reaktionsgemisch keine unerwünschten Nebenreaktionen eingehen, also inert sein. Ausserdem soll es so gewählt werden, dass sein Azeotrop leichter siedet als der zur Acetalisierung eingesetzte Alkohol. (Auch bei dem vorher erläuterten Fall, bei dem der Alkohol selbst als Schleppmittel benutzt wird, siedet sein Azeotrop leichter als der Alkohol selbst.) Bei Einsatz von Alkoholen wie Ethanol, Isopropanol und tert.-Butanol zur Acetalisierung wird man im allgemeinen den Alkohol auch als Schleppmittel benutzen, da die entsprechenden Azeotrope unterhalb von 90°C sieden. Hier ist die Verwendung eines zusätzlichen inerten Schleppmittels zwar auch möglich, bedeutet aber einen überflüssigen Aufwand.

Ein weiterer Gegenstand der vorliegenden Erfindung ist demgemäss ein Verfahren zur Herstellung von Glyoxylsäurehemiacetalestern, dadurch gekennzeichnet, dass man Glykolsäureester in der Gasphase katalytisch zu Glyoxylsäureestern oxydehydriert und diese dann mit einem Alkohol umsetzt, wobei man das bei der Oxydehydrierung gebildete gasförmige Reaktionsgemisch zusammen mit einem Überschuss Alkohol und einem inerten Schleppmittel, dessen Wasser-Azeotrop unterhalb von 90°C und unterhalb des eingesetzten Alkohols siedet, in eine Destillationskolonne einleitet und dann das Reaktionswasser zusammen mit anderen Leichtsiedern und dem Schleppmittel über Kopf destilliert, wobei der Glyoxylsäurehemiacetalester und der überschüssige Alkohol als Sumpfprodukt anfallen.

Vorzugsweise verwendet man ein inertes Schleppmittel, dessen Wasser-Azeotrop unterhalb von 80°C und unterhalb des eingesetzten Alkohols siedet.

Wenn ein inertes Schleppmittel ein binäres Azeotrop mit Wasser bildet, so bildet sich im allgemeinen auch ein ternäres Azeotrop aus Wasser, dem eingesetzten Alkohol und dem Schleppmittel, so dass auch etwas von dem Alkohol über Kopf geht.

Aus dem Kopfprodukt kann das Schleppmittel und der Alkohol nach bekannten Methoden, z.B. durch Destillation oder durch Phasentrennung gewonnen und zurückgeführt werden.

Als Alkohole eignen sich für die Hemiacetalherstellung prinzipiell alle geradkettigen oder verzweigten aliphatischen Alkohole mit 1 bis 8 C-Atomen. Bevorzugt sind gesättigte primäre und sekundäre Alkohole mit 1 bis 8 C-Atomen. Besonders bevorzugt sind Methanol, Ethanol, n-Propanol und n-Butanol.

Bei dem erfindungsgemässen Verfahren setzt man pro Mol Glyoxylsäureester im allgemeinen 1-30 Mol Alkohol ein. Wird der Alkohol auch als Schleppmittel benutzt, so beträgt die Alkoholmenge vorzugsweise 2-20 Mol pro Mol Ester. Bei Einsatz eines zusätzlichen inerten Schleppmittels beträgt die Alkoholmenge vorzugsweise 2-10 Mol pro Mol Ester.

Geeignete inerte Schleppmittel stammen aus der Reihe der aliphatischen und aromatischen Kohlenwasserstoffe, die Heteroatome enthalten können, wie $CH_2Cl_2$, $CHCl_3$, $CCl_4$, n-Pentan, n-Hexan, Cyclohexan und Benzol. Die Menge an Schleppmittel muss natürlich ausreichen, um das Reaktionswasser über Kopf zu entfernen.

Das gasförmige Gemisch aus dem Oxydehydrierungsreaktor wird im allgemeinen in eines der beiden unteren Drittel der Destillationskolonne (inklusive Sumpf) eingeleitet, vorzugsweise in das unterste Drittel (inklusive Sumpf). Der Alkohol wird im allgemeinen oberhalb dieses Gasstroms in die Kolonne eingeleitet. Ebenso das inerte Schleppmittel, falls eines verwendet wird.

Glyoxylsäureester und deren Hemiacetale sind aufgrund ihrer hohen Reaktivität wertvolle Ausgangs- und Zwischenprodukte für eine Reihe von Synthesen pharmazeutisch wirksamer Verbindungen, wie z.B. Allantoin, substituierter Glycine oder Alkaloide (wie z.B. Tetrahydroisochinolin-Alkaloide).

Die nachfolgende Beispiele sollen die Erfindung näher erläutern.

*Beispiel 1*

Die Oxydehydrierung des Glykolsäuremethylesters wurde bei 320°C mit 22,5 ml des im Beispiel 1 der US-PS 4 340 748 beschriebenen Katalysators durchgeführt. Die stündlich erzeugten Mengen der Hauptkomponenten im Reaktoraustrag sind in der Tabelle 1 wiedergegeben:

TABELLE 1

| Gasphasenbestandteile | mmol/Stunde |
|---|---|
| Glyoxylsäuremethylester | 112,0 |
| Glykolsäuremethylester | 2,6 |
| Wasser | 123,2 |
| Methanol | 7,8 |
| Stickstoff | 6430,0 |
| Ameisensäure | 2,4 |

Der Reaktoraustrag enthielt ausserdem z.B. $O_2$, CO, $CO_2$, HCHO und $HCOOCH_3$. Die Ausbeute an Glyoxylsäuremethylester und der Umsatz betrugen 86 bzw. 98%.

Der gasförmige Reaktoraustrag wurde zusammen mit einem Gemisch aus Methanol und Chloroform kontinuierlich in den Sumpf einer Destillationskolonne (Länge: 300 mm, Durchmesser: 12 mm, Füllung: Braunschweiger Wendeln) eingeleitet. Die stündlich eingeleiteten Mengen an Methanol und Chloroform betrugen 30 g bzw. 150 g. Der Sumpfinhalt wurde kräftig gerührt und die Sumpftemperatur betrug 30°C. Die leichte Flüchtigkeit der Sumpfkomponenten war durch die Wirkung des starken Gasstromes gewährleistet. Ein Gemisch aus Chloroform, Methanol, Wasser und anderen Leichtsiedern wurde über Kopf entfernt. Ein Teil des Sumpfgemisches wurde kontinuierlich während des Versuches abgelassen. Nach einer vierstündigen Versuchsdauer wurde der restliche Sumpfinhalt zum schon abgelassenen Sumpfprodukt gegeben. Dieses gesammelte Sumpfgemisch enthielt folgende Produkte:

HO
$>CHCOOCH_3$ 444,2 mmol
$CH_3O$

$CH_3O$
$>CHCOOCH_3$ nicht nachweiswar
$CH_3O$

$HOCH_2COOCH_3$ 10,1 mmol

HO
$>CHCOOCH_3$ Spuren
$CH_3OOC\ CH_2O$

Glyoxylsäuremethylester 2,7 mmol

$H_2O$ 22,3 mmol

Der Nachweis des Glyoxylsäureesters und seines Hemiacetals erfolgte über $C^{13}$-NMR-Spektroskopie. Ausserdem wurden gaschromatographische Analysenmethoden angewendet.

Der Glyoxylsäuremethylester wurde also fast quantitativ zum Methylhemiacetal umgewandelt, während 95,5% des Reaktionswassers über Kopf entfernt wurden.

*Beispiel 2*

Die Oxydehydrierungsbedingungen und Gasphasenzusammensetzung waren wie im Beispiel 1. Der gasförmige Reaktoraustrag wurde zusammen mit einem Gemisch aus 2-Butanol (60 g/h) und n-Hexan (100 g/h) in den Sumpf der Kolonne eingeleitet. Die Temperatur im kräftig gerührten Sumpf betrug 35°C. Ein Gemisch aus 2-Butanol, n-Hexan, Wasser und anderen Leichtsiedern wurde über Kopf entfernt und ein Teil des Sumpfgemisches wurde kontinuierlich abgelassen. Nach vierstündiger Versuchsdauer enthielt das gesammelte Sumpfgemisch folgende Produkte (R' = sek. Butyl):

HO
$>CHCOOCH_3$ 434,2 mmol
R'O

R'O
$>CHCOOCH_3$ nicht nachweisbar
R'O

HO
>CHCOOR' nicht nachweisbar
R'O

HO
$>CHCOOCH_3$ 13,2 mmol
$CH_3O$

$HOCH_2COOCH_3$ 5,1 mmol

Glyoxylsäuremethylester nicht nachweisbar

$H_2O$ 24,8 mmol

Vollacetale und Umesterungsprodukte waren also nicht nachweisbar.

*Beispiel 3*

Die Oxydehydrierungsbedingungen und Gasphasenzusammensetzung waren wie in Beispiel 1. Der gasförmige Reaktoraustrag wurde zusammen mit Ethanol (88 g/h) in den Sumpf der Kolonne geleitet. Hierbei diente Ethanol als Reaktionspartner und Schleppmittel. Die Temperatur im kräftig gerührten

Sumpf betrug 58 °C. Ein Gemisch aus Ethanol, Wasser und anderen Leichtsiedern wurde über Kopf entfernt und ein Teil des Sumpfgemisches wurde kontinuierlich abgelassen. Nach vierstündiger Versuchsdauer enthielt das gesammelte Sumpfgemisch folgende Produkte:

| | |
|---|---|
| HO<br>$>CHCOOCH_3$<br>$C_2H_5O$ | 418,1 mmol |
| $C_2H_5O$<br>$>CHCOOCH_3$<br>$C_2H_5O$ | nicht nachweisbar |
| HO<br>$>CHCOOC_2H_5$<br>$C_2H_5O$ | 16,3 mmol |
| HO<br>$>CHCOOCH_3$<br>$CH_3O$ | 7,2 mmol |
| $HOCH_2COOCH_3$ | 4,6 mmol |
| Glyoxylsäuremethylester | 2,3 mmol |
| $H_2O$ | 21,4 mmol |

*Beispiel 4*

Die Oxydehydrierungsbedingungen und Gasphasenzusammensetzung waren wie in Beispiel 1. Der gasförmige Reaktorenaustrag wurde zusammen mit einem Gemisch aus n-Octanol (73 g/h) und Cyclohexan (50 g/h) in den Sumpf der Kolonne eingeleitet. Die Temperatur im kräftig gerührten Sumpf betrug 61 °C. Ein Gemisch aus n-Octanol, Wasser, Cyclohexan und anderen Leichtsiedern wurde über Kopf entfernt, und ein Teil des Sumpfgemisches wurde kontinuierlich abgelassen. Nach vierstündiger Versuchsdauer enthielt das gesammelte Sumpfgemisch folgende Produkte:

| | mit R′ = n-Octyl |
|---|---|
| HO<br>$>CHCOOCH_3$<br>R′O | 439,5 mmol |
| R′O<br>$>CHCOOCH_3$<br>R′O | nicht nachweisbar |
| $CH_3O$<br>$>CHCOOCH_3$<br>HO | Spuren |
| HO<br>>CHCOOR′<br>R′O | nicht nachweisbar |
| $HOCH_2COOCH_3$ | 7,8 mmol |
| Glyoxylsäuremethylester | 6,1 mmol |
| $HO_2$ | 16,7 mmol |

**Patentansprüche**

1. Verfahren zur Herstellung von Glyoxylsäurehemiacetalestern, dadurch gekennzeichnet, dass man Glykolsäureester in der Gasphase katalytisch zu Glyoxylsäureestern oxydehydriert und diese dann mit einem Alkohol umsetzt, wobei man das bei der Oxydehydrierung gebildete gasförmige Reaktionsgemisch zusammen mit einem Überschuss eines als Schleppmittel geeigneten Alkohols in eine Destillationskolonne einleitet und dann das Reaktionswasser zusammen mit anderen Leichtsiedern und einem Teil des überschüssigen Alkohols über Kopf destilliert, wobei der Glyoxylsäurehemiacetalester und der Rest des überschüssigen Alkohols als Sumpfprodukt anfällt.

2. Verfahren nach Anspruch 1, dadurch gekennzeichnet, dass der Katalysator für die Oxydehydrierung mindestens eines der Elemente Ag, V, Mo, Cu, Au, Sn, Sb, Bi und P auf einem Träger enthält.

3. Verfahren nach Anspruch 1 oder 2, dadurch gekennzeichnet, dass man gesättigte primäre oder sekundäre Alkohole mit 1 bis 8 C-Atomen einsetzt.

4. Verfahren zur Herstellung von Glyoxylsäurehemiacetalestern, dadurch gekennzeichnet, dass man Glykolsäureester in der Gasphase katalytisch zu Glyoxylsäureestern oxydehydriert und diese dann mit einem Alkohol umsetzt, wobei man das bei der Oxydehydrierung gebildete gasförmige Reaktionsgemisch zusammen mit einem Überschuss Alkohol und einem inerten Schleppmittel, dessen Wasser-Azeotrop unterhalb von 90 °C und unterhalb des eingesetzten Alkohols siedet, in eine Destillationskolonne einleitet und dann das Reaktionswasser zusammen mit anderen Leichtsiedern und dem Schleppmittel über Kopf destilliert, wobei der Glyoxylsäurehemiacetalester und der überschüssige Alkohol als Sumpfprodukt anfallen.

5. Verfahren nach Anspruch 4, dadurch gekennzeichnet, dass der Katalysator für die Oxydehydrierung mindestens eines der Elemente Ag, V, Mo, Cu, Au, Sn, Sb, Bi und P auf einem Träger enthält.

6. Verfahren nach Anspruch 4 oder 5, dadurch gekennzeichnet, dass man gesättigte primäre oder sekundäre Alkohole mit 1 bis 8 C-Atomen einsetzt.

7. Verfahren nach einem der Ansprüche 4 bis 6, dadurch gekennzeichnet, dass man ein inertes Schleppmittel verwendet, dessen Wasser-Azeotrop unterhalb von 80 °C und unterhalb des eingesetzten Alkohols siedet.

8. Verfahren nach einem der Ansprüche 4 bis 7, dadurch gekennzeichnet, dass man als inertes Schleppmittel $CH_2Cl_2$, $CHCl_3$, $CCl_4$, n-Pentan, n-Hexan, Cyclohexan oder Benzol verwendet.

**Claims**

1. A process for the preparation of glyoxylic hemiacetal esters, which comprises catalytic oxydehydrogenation of glycolic esters in the gas phase to give glyoxylic esters and then reaction of the latter with an alcohol, the gaseous reaction mixture formed in the oxydehydrogenation being introduced together with an excess of an alcohol which is suitable as an

eintraining agent into a distillation column, and then the water of reaction being distilled overhead with other low-boilers and part of the excess alcohol, the glyoxylic hemiacetal ester and the remainder of the excess alcohol resulting as the bottom product.

2. The process as claimed in claim 1, wherein the catalyst for the oxydehydrogenation contains at least one of the elements Ag, V, Mo, Cu, Au, Sn, Sb, Bi and P on a support.

3. The process as claimed in claim 1 or 2, wherein saturated primary or secondary alcohols having 1 to 8 carbon atoms are used.

4. A process for the preparation of glyoxylic hemiacetal esters, which comprises catalytic oxydehydrogenation of glycolic esters in the gas phase to give glyoxylic esters, and then reaction of the latter with an alcohol, the gaseous reaction mixture formed in the oxydehydrogenation being introduced, together with an excess of alcohol and an inert entraining agent whose azeotrope with wather boils below 90°C and below the alcohol which is used, into a distillation column and then distilling overhead the water of reaction together with other low-boilers and the entraining agent, the glyoxylic hemiacetal ester and the excess alcohol resulting as the bottom product.

5. The process as claimed in claim 4, wherein the catalyst for the oxydehydrogenation contains at least one of the elements Ag, V, Mo, Cu, Au, Sn, Sb, Bi and P on a support.

6. The process as claimed in claim 4 or 5, wherein saturated primary or secondary alcohols having 1 to 8 carbon atoms are used.

7. The process as claimed in one of claims 4 to 6, wherein use is made of an inert entraining agent whose azeotrope with water boils below 80°C and below the alcohol which is used.

8. The process as claimed in one of claims 4 to 7, wherein the inert entraining agent used is $CH_2Cl_2$, $CHCl_3$, $CCl_4$, n-pentane, n-hexane, cyclohexane or benzene.

**Revendications**

1. Procédé pour préparer des hémiacétals esters de l'acide glyoxylique, procédé caractérisé en ce qu'on oxydéshydrogène catalytiquement des esters de l'acide glycolique, en phase gazeuse, pour les convertir en esters de l'acide glyoxylique, puis on fait réagir ceux-ci avec un alcool, cela en introduisant le mélange réactionnel gazeux formé au cours de l'oxydéshydrogènation, avec un excès d'un alcool approprié comme agent d'entraînement, dans une colonne de distillation, puis en distillant par la tête l'eau réactionnel avec d'autres substances à bas point d'ébullition et une partie de l'excès d'alcool, l'hémiacétal ester de l'acide glyoxylique et le reste de l'excès d'alcool étant obtenus comme produit de bas de colonne.

2. Procédé selon la revendication 1, caractérisé en ce que le catalyseur d'oxydéshydrogènation contient, sur un support, au moins l'un des éléments suivants: Ag, V, Mo, Cu, Au, Sn, Sb, Bi et P.

3. Procédé selon la revendication 1 ou 2, caractérisé en ce qu'on utilise des alcools saturés primaires ou secondaires qui contiennent de 1 à 8 atomes de carbone.

4. Procédé de préparation d'hémiacétals esters de l'acide glyoxylique, procédé caractérisé en ce qu'on oxydéshydrogène catalytiquement, en phase gazeuse, des esters de l'acide glycolique pour les transformer en esters de l'acide glyoxylique, puis on fait réagir ceux-ci avec un alcool, cela en introduisant dans une colonne de distillation le mélange réactionnel gazeux formé lors de l'oxydéshydrogènation, avec un excès d'alcool et un agent d'entraînement inerte dont l'azéotrope avec l'eau bout à une température inférieure à 90°C et inférieure à la température d'ébullition de l'alcool mis en jeu, puis en distillant par la tête l'eau réactionnelle avec d'autres substances à bas point d'ébullition et l'agent d'entraînement, l'hémiacétal ester glyoxylique et l'excès d'alcool étant alors obtenu comme produit de bas de colonne.

5. Procédé selon la revendication 4, caractérisé en ce que le catalyseur d'oxydéshydrogènation contient, sur un support, au moins l'un des élements suivants: Ag, V, Mo, Cu, Au, Sn, Sb, Bi et P.

6. Procédé selon la revendication 4 ou 5, caractérisé en ce qu'on utilise des alcools saturés primaires ou secondaires qui contiennent de 1 à 8 atomes de carbone.

7. Procédé selon l'une quelconque des revendications 4 à 6, caractérisé en ce qu'on utilise un agent d'entraînement inerte dont l'azéotrope avec l'eau bout à une température inférieure à 80°C et inférieure à la température d'ébullition de l'alcool mis en jeu.

8. Procédé selon l'une quelconque des revendications 4 à 7, caractérisé en ce qu'on utilise, comme agent d'entraînement inerte, $CH_2Cl_2$, $CHCl_3$, $CCl_4$, le n-pentane, le n-hexane, le cyclohexane ou le benzène.